(19) Europäisches Patentamt
European Patent Office
Office européen des brevets

(11) Publication number: **0 036 534**
**B1**

(12) # EUROPEAN PATENT SPECIFICATION

(45) Date of publication of patent specification: **23.09.87**

(21) Application number: **81101642.7**

(22) Date of filing: **06.03.81**

(51) Int. Cl.[4]: **A 61 K 31/62,** A 61 K 31/60, A 61 K 31/66, A 61 K 31/43, A 61 K 31/545, A 61 K 45/06, A 61 K 47/00 // (A61K31/66, 31:60, 31:545, 31:43, 31:19)

(54) **An orally administrable drug form comprising a beta-lactam antibiotic and an adjuvant.**

(30) Priority: **07.03.80 US 128099**

(43) Date of publication of application:
**30.09.81 Bulletin 81/39**

(45) Publication of the grant of the patent:
**23.09.87 Bulletin 87/39**

(84) Designated Contracting States:
**AT BE CH DE FR GB IT LI LU NL SE**

(56) References cited:
**EP-A-0 003 618**
**GB-A-1 036 194**
**US-A-3 676 434**

**CHEMICAL ABSTRACTS, vol. 75, no. 9, August 30, 1971, page 160, abstract 60308q, COLUMBUS, OHIO (US);D. KAPLAN: "Synergistic effect of combinations of sodium salicylate and antibiotics on strains of Escherichia coli and Staphylococcus aureus"**

**D. KAPLAN, CHEMOTHERAPY (1971), 16, pages 252-258**

(73) Proprietor: **INTERx RESEARCH CORPORATION**
**2201 West 21st Street**
**Lawrence Kansas 66044 (US)**

(72) Inventor: **Higuchi, Takeru**
**2811 Schwarz Road**
**Lawrence Kansas 66044 (US)**
Inventor: **Nishihata, Toshiaki**
**2428 Redbud Lane**
**Lawrence Kansas 66044 (US)**

(74) Representative: **Abitz, Walter, Dr.-Ing. et al**
**Abitz, Morf, Gritschneder P.O. Box 86 01 09**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 036 534 B1**

**Description**

Background of the Invention

*Field of the Invention*

The present invention relates to the oral delivery of β-lactam antibiotics which by this route are poorly absorbed and more especially to the enhancement of this delivery by formulations which contain a hydroxyaryl or hydroxyaralkyl acid or salt thereof of formula I.

As employed in this application, the term "β-lactam antibiotics" refers to those antibiotics which contain the β-lactam moiety and include the penicillins, cephalosporins and related chemical species.

*Description of the Prior Art*

It is well known in this art that the β-lactam antibiotics which have the common structural feature, a four-member lactam ring, have been described as the most important class of therapeutic agents to combat gram-positive and gram-negative infections. Inspite of their great popularity, it is also well known that a number of the β-lactam antibiotics such as the penicillins, penicillin G, methacillin and carbenicillin, and the cephalosporins, cefazolin, cephapirin, cephaloridine, cephalothin, cephanone, cefamandole, cefaparole, cefoxitin, cephacetrile, cefmetazole, cefuroxime, cefotaxime, cetoperazone, and latamoxef show poor oral activity.

The penicillins, which were introduced a number of years ago, suffer from two major disadvantages: poor activity against resistant organisms and lack of oral activity which is due to its inherent instability to gastric acid. The acid instability was partially overcome by the discovery of more acid stable penicillins such as penicillin V, which permit the penicillin to be absorbed with less degradation and hence produce higher blood levels of the active therapeutic agent. However, inspite of these chemical modifications to produce acid stable, orally effective penicillins, many of the penicillins in clinical use, such as penicillin G, methacillin, carbenicillin and ticarcillin, cannot be administered by the oral route. Even the newer esters of carbenicillin, carfecillin and carindacillin are only 40% absorbed and give low blood levels of carbenicillin.

The second major problem with penicillin antibiotics is their lack of activity against resistant strains of bacteria which produce the degrading enzyme, penicillinase. The earlier semisynthetic penicillins, such as methicillin, oxacillin, cloxacillin, dicloxacillin, flucloxacillin and nafcillin, were developed to overcome this problem. This class of penicillins were resistant to the penicillinase enzyme and were active against those resistant organisms which produce the enzyme. However, these compounds were less active than their parents and in particular showed poor activity against the important gram-negative organisms.

The second class of β-lactam antibiotics, the cephalosporins, were developed because they, unlike the penicillins, were very active against both the penicillinase producing gram-positive bacteria and the gram-negative bacteria. However, lack of oral activity, an almost universal characteristic of the cephalosporins, is shared by the parent molecule, cephalosporin C, and most of the newer semisynthetic analogues. This lack of oral activity was totally unexpected because the cephalosporins, unlike the penicillins, are stable in gastric acid. Since the molecule is excreted unchanged in the feces, it is apparent that the lack of oral activity is due to poor absorption and is probably caused by the polar nature of the cephalosporin nucleus. Through chemical modification a number of orally effective β-lactam antibiotics were prepared, such as cephalexin, cephradine, cephaloglycin, cefadioxil and cefaclor, which have been shown to be greater than 80% absorbed. Unfortunately, they are much less active *in vitro* than the clinically injectable cephalosporins. It is clear that this chemical modification has produced an orally active antibiotic whose antibacterial properties are inferior to those of the currently used injectable cephalosporins.

Thus, there exists a clear·and present need for a novel method to enhance the oral absorption of the non-orally effective β-lactam antibiotics. Said method would permit the oral use of the clinically important β-lactam antibiotics whose use is presently limited to intramuscular and intravenous administration.

*Summary of the Invention*

Accordingly, a major object of this invention is to provide a class of agents which enhance the oral absorption of β-lactam antibiotics.

Another object is to provide a process utilizing said class of agents to enhance the oral absorption of β-lactam antibiotics.

Another object is to provide a stable drug form utilizing said class of agents which when administered orally will provide increased blood levels of the therapeutic agent.

Other objects, features and advantages of the invention will be apparent to those skilled in the art from the detailed description of the invention which follows.

All of the foregoing objects are readily attained by providing an orally administerable drug form comprising a therapeutically effective amount of a β-lactam antibiotic and an adjuvant having the following formula I

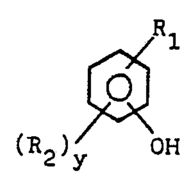

,

wherein

$R_1$ is

$$-CO_2H,\ -(CH_2)-COOH,\ -\underset{\displaystyle OH}{\overset{\displaystyle H}{\underset{|}{\overset{|}{C}}}}-CO_2H,\ -SO_3H,$$

or a pharmaceutically acceptable salt thereof,

$R_2$ is selected from OH, H, an alkoxy radical having 1—10 carbon atoms, an alkyl radical having 1—10 carbon atoms, a halo radical or a tri-halo-alkyl radical, wherein the alkyl group has 1—5 carbon atoms, and

y is an integer of 1 or 2, said adjuvant being present in said drug form in a sufficient amount to be effective in enhancing the oral absorption rate of said β-lactam antibiotic.

Preferred adjuvants are those having the formula:

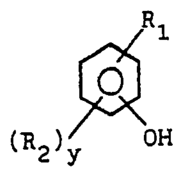 ,

wherein $R_2$ is OH, H, methoxy, ethoxy, butoxy, octyloxy, methyl, isopropyl, ethyl, t-butyl, n-butyl, t-octyl, a halo radical, or trifluoromethyl.

Specific adjuvants useful in our method and drug forms for enhancing oral absorption of β-lactam antibiotics include salicylic acid, resorcylic acid, and gentisic acid. Other hydroxyaryl acids, such as the compounds as mentioned at the end of this specification, have similar useful adjuvant activity in our process. Such adjuvants are not considered novel *per se* and may be prepared by techniques known to those skilled in the art.

The amount of hydroxyaryl or hydroxyaralkyl acid or salt thereof used in our method and drug forms may vary over a wide range; in general, the identity and the amount of the hydroxyaryl or hydroxyaralkyl acids or salt thereof is used in connection with the drug in order to be effective in enhancing the absorption rate of the drug from the gastrointestinal compartment into the bloodstream. The effectiveness of the hydroxyaryl or hydroxyaralkyl acid or salt thereof becomes significant at local concentration exceeding 0.01% at the absorption site. Their use at a dosage whereby their concentration at the absorption site exceeds 5% is not recommended because of the local irritating effect on the tissue.

The β-lactam antibiotics whose enhanced oral delivery is a subject of the present invention encompass both the penicillins, penicillin G, methacillin, carbenicillin and ticaricillin, and the cephalosporins, cephalosporin C, cefazolin, cephapirin, cephaloridine, cephalothin, cephanone, cefamandole, cefaparole, cefoxitin, cephacetrile, cefmetazole, cefoxitin, cefuroxime, cefotaxime, T—1551, and the oxacephalosporin, S—6059. The quantity of β-lactam antibiotic necessary for preparing the drug form could vary over a wide range but would normally be regulated by that quantity necessary to comprise the therapeutically effective unit dosage.

Example 1

The sodium salt of cefmetazole (50 mg/kg) and sodium salicylate (200 mg/kg) dissolved in water were given to mice by gavage. As a control, mice were given an equal dose of the sodium salt of cefmetazole in water without the added sodium salicylate. The mice were placed in individual metabolism cages and their urines collected after 24 hours.

The urine samples were acidified to pH 2.0 with 1N phosphoric acid. Acidified samples were applied to 100—200 mesh XAD.2 columns (1.5 ml), and the column washed with $H_2O$. Cefmetazole was eluted with 2 ml methanol and measured by high pressure liquid chromatography which was carried out using an Altex liquid chromatograph equipped with a dual wavelength recorder (254 nm and 280 nm). The column was a LiChrosorb 10 RP—18 (length = 25 cm., internal diameter — 4.6 mm) obtained from Chrompack, Whittier, CA. All assays were done at ambient temperatures. A 3 cm guard column of RP—18 column material (Rheodyne, Inc., Berkeley, CA) was also used. The mobile phase consisted of 30% tetrahydrofuran, $7.5 \times 10^{-4}$ M tetra-n-hexyl ammonium perchlorate, and 70% $H_2O$. The flow rate was 2 ml/min and the pressure less than 138 bar (2000 psi). Concentrations of cefmetazole were determined by measuring peak height at 254 nm and evaluating on the basis of standard curves run under identical conditions. The results are shown in Table I.

TABLE I
Urine Levels of Cefmetazole

| | Number of Mice | Percent of Dose in Urine |
|---|---|---|
| Cefmetazole 50 mg/kg + sodium salicylate 200 mg/kg | 22 | 70.8 ± 11.9 |
| Cefmetazole 50 mg/kg | 8 | 9.1 ± 7.5 |
| Standard deviation | | |

In like manner the following combinations of other β-lactams and hydroxyaryl acids were also found to enhance the oral absorption of the corresponding antibiotic.

| Example | β-lactam Antibiotic | Hydroxyaromatic Acid |
|---|---|---|
| 2 | penicillin G. | salicylic acid |
| 3 | methacillin | sodium salicylate |
| 4 | carbenicillin | gentisic acid |
| 5 | carbenicillin | sodium salicylate |
| 6 | carbenicillin | resorcylic acid |
| 7 | cephalosporin C | salicylic acid |
| 8 | cefazolin | sodium salicylate |
| 9 | cefuroxine | gentisic acid |
| 10 | cephaprin | homovanillic acid |
| 11 | cephaloridine | sodium salicylate |
| 12 | cephmetazole | homovanillic acid |
| 13 | cephanone | salicylic acid |
| 14 | cefmetazole | salicylic acid |
| 15 | cefaparole | sodium salicylate |
| 16 | cephacetrile | gentisic acid |
| 17 | cefoxitin | resorcylic acid |
| 18 | cefuroxime | sodium salicylate |
| 19 | T—1551 | homovanillic acid |
| 20 | cefamandole | gentisic acid |
| 21 | cephalothin | salicylic acid |

The drug forms of this invention are suitably administered in oral dosage form, such as by tablet or capsule, by combining the β-lactam antibiotic in a therapeutic amount and the hydroxyaromatic acid or salt thereof in sufficient quantity to be effective to enhance oral delivery with any oral pharmaceutically acceptable inert carrier, such as lactose, starch (pharmaceutical grade), dicalcium phosphate, calcium sulfate, Kaolin, mannitol and powdered sugar. In order to reduce the irritation in the stomach, the preferred dose form of the hydroxyaromatic acid should be a pharmaceutically acceptable salt and the drug form should be designed to release the β-lactam antibiotic and the hydroxyaromatic acid salt beyond the

pylorus. In addition, when required, suitable binders, lubricants, disintegrating agents, and coloring agents can also be added. Typical binders include, without limitation, starch, gelatin, sugars such as sucrose, molasses, and lactose, natural and synthetic gums, such as acacia, sodium alginate, extract of Irish moss, carboxymethylcellulose, methylcellulose, and polyvinylpyrrolidone, polyethylene glycol, ethylcellulose and waxes. Typical lubricants for use in these dosage forms can include, without limitation, boric acid, sodium benzoate, sodium acetate, sodium chloride, leucine and polyethylene glycol. Suitable disintegrators can include, without limitation, starch, methylcellulose, agar, bentonite, cellulose and wood products, alginic acid, guar gum, citris pulp, carboxymethylcellulose, and sodium lauryl sulfate. Optionally, if desired, a conventionally, pharmaceutically acceptable dye can be incorporated into the oral dosage unit form, e.g., any of the standard FD & C dyes.

## Example 2

Preparation of Sodium 2-hydroxy-5-methoxy benzenesulfonate

p-Methoxyphenol (12.4 g) was dissolved in chloroform (100 ml) and cooled in ice. Chlorosulfonic acid (11.6 g) was added dropwise to the stirred reaction mixture. The cooling bath was removed after the addition and stirring continued for 24 hours at room temperature. The chloroform was then evaporated off and the residue was vacuum dried to a hygroscopic light brown solid weighing 20.5 g which was 2-hydroxy-5-methoxy-benzenesulfonic acid.

NMR ($CDCl_3$) 3.73 (3H, s, $OC\underline{H}_3$), 6.8—7.2 (3H, m, aromatic $\underline{H}$), and 9.86 (2H, broad s, $O\underline{H}$ and $SO_3\underline{H}$). IR (film) 3500—2900, 1512, 1470, 1229, 1198, 996, 938 $cm^{-1}$.

The above sulfonic acid (10 g) was dissolved in water (10 ml) and poured into 75 ml of saturated sodium chloride solution. A white solid separated immediately. It was filtered and dried. Crystallization from water gave the pure sodium salt of 2-hydroxy-5-methoxybenzenesulfonic acid (6.6 g).

NMR ($D_2O$) 3.83 (3H, s, $OC\underline{H}_3$), 7.05 and 7.33 (3H, multiplets, aromatic). IR (KBr) 3260, 1518, 1440, 1300, 1280, 1240, 1210, 1905, 1045 $cm^{-1}$.

## Example 3

Typical preparation of enteric-coated tablets containing adjuvant.

*300 mg Cefoxitin Tablets*

| Ingredient | | Amount per Tablet |
|---|---|---|
| Cefoxitin (sodium salt) | | 300 mg |
| Sodium 5-methoxysalicylate | | 300 mg |
| Microcrystalline cellulose | | 70 mg |
| Magnesium stearate | | 30 mg |
| | Total | 700 mg |

The cefoxitin sodium salt was ground, passed through a 40 mesh screen, mixed with the sodium 5-methoxysalicylate, ½ the magnesium stearate and slugged with 1.27 cm (½") flat punches. The slugs were broken up and passed through a 40 mesh screen, mixed with microcrystalline cellulose and the remaining magnesium stearate. The material was tableted using 1.11 cm (7/16") deep concave punches to give tablets of 10 Kg hardness.

Coating

The tablets were coated with 15 mg pre-coat and 34 mg enteric coat according to the coating procedure described below.

*Entering Coating Procedure*

Tablets or capsules were placed in a coating pan containing baffles to provide adequate tumbling. A small amount of the coating solution was applied using an air sprayer and the solvents evaporated with a warm air supply directed into the coating pan. This procedure was repeated until the desired amount of coating material was applied. The amount of coating material was determined from the weight gain of a representative group of tablets.

Coating Solutions

*Pre-coat:* A film of hydroxypropylmethylcellulose was applied to the tablets followed by an enteric coating.

*Enteric coat:* A film of hydroxypropylmethylcellulosephthalate was applied.

*Solutions:* A 5% by weight solution of hydroxypropylmethylcellulose and a 10% by weight solution of hydroxypropylmethylcellulosephthalate in ethanol:methylene chloride (1:1 by weight) were used as the coating solutions.

Example 4

Following the procedure of Example III for the preparation of enteric-coated tablets and using equivalent quantities of ingredients, the following compounds can be substituted for cefoxitin which is (6R - cis) - 3 - [[(aminocarbonyl)oxy]methyl] - 7 - methoxy - 8 - oxo - 7[(2 - thienylacetyl)amino] - 5 - thia - 1 - azabicyclo[4.2.0]oct - 2 - ene - 2 - carboxylic acid.

1) Cephamandole which is 7 - [(hydroxyphenylacetyl)amino] - 3 - [[(1 - methyl - 1H - tetrazol - 5 - yl)thio]methyl] - 8 - oxo - 5 - thia - 1 - azabicyclo[4.2.0]oct - 2 - ene - 2 - carboxylic acid.

2) Amoxicillin which is 6 - [[amino(4 - hydroxyphenyl)acetyl]amino] - 3,3 - dimethyl - 7 - oxo - 4 - thia - 1 - azabicyclo[3.2.0]heptane - 2 - carboxylic acid.

3) N - formimidoyl thienamycin monohydrate.

Also the following adjuvants may be used in combination with any of the drugs or compounds shown in Examples 1, 3 or 4 in appropriate ratios.

The adjuvants may be chosen from the following salts or the corresponding acids:

Sodium 5-methoxysalicylate
Sodium salicylate
Sodium homovanilate
Sodium 2,5-dihydroxybenzoate
Sodium 2,4-dichydroxybenzoate
Sodium 3,4-dihydroxymandelate
Sodium 3-methoxy-4-hydroxymandelate
Sodium 5-methoxy-2-hydroxyphenylsulfonate
Sodium 3-methylsalicylate
Sodium 5-methylsalicylate
Sodium 5-tert-octylsalicylate
Sodium 3-tert-butyl-6-methylsalicylate
Sodium 3,5-diisopropylsalicylate
Sodium 3-tert-butyl-5-methylsalicylate
Sodium guaicolsulfonate
Sodium 5-bromosalicylate
Sodium 3,5-dibromosalicylate
Sodium 5-iodosalicylate
Sodium 3,5-diiodosalicylate
Sodium 2-hydroxyphenylacetate
Sodium 5-trifluoromethyl-2-hydroxybenzoate
Sodium 3-methoxysalicylate
Sodium 5-octyloxysalicylate
Sodium 5-butoxysalicylate
Sodium 3,4-dihydroxyphenylacetate
Sodium 5-chlorosalicylate
Sodium 3,5-dihydroxybenzoate
Sodium 2-hydroxy-3-methoxybenzoate

Any skilled artisan concerned with the subject matter of this invention, can prepare these oral dosage forms by simply referring to the oral dosage form preparatory procedure outlined in REMINGTON'S PHARMACEUTICAL SCIENCES, Fifteenth Edition (1975), pages 1576 through 1617 inclusive.

**Claims for the Contracting States: BE CH DE FR GB IT LI LU NL SE**

1. An orally administerable drug form comprising a therapeutically effective amount of β-lactam antibiotic and an adjuvant having the following formula I

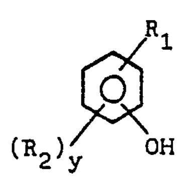 ,

wherein

$R_1$ is

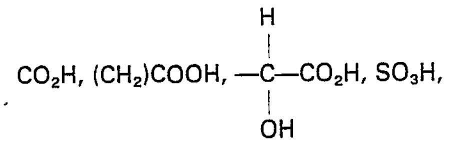

or a pharmaceutically acceptable salt thereof,

$R_2$ is OH, H, an alkoxy radical having 1—10 carbon atoms, an alkyl radical having 1—10 carbon atoms, a halo radical, or a tri-halo alkyl radical, wherein the alkyl group has 1—5 carbon atoms, and

y is an integer of 1 or 2, said adjuvant being present in said drug form in sufficient amount to be effective in enhancing the oral absorption rate of said β-lactam antibiotic.

2. The drug form of Claim 1 wherein said β-lactam antibiotic is a penicillin, a cephalosporin or thienamycin.

3. The drug form of Claim 2 wherein said penicillin is penicillin G, methacillin, carbenicillin, ticaricillin or amoxicillin.

4. The drug form of Claim 2 wherein said cephalosporin is cephalosporin C, cefazolin, cephapirin, cephaloridine, cephalothin, cephapirin, cephanone, cefamandole, cefaparole, cefoxitin, cephacetrile, cefmetazole, cefuroxime, cefotaxime, cefoperazone or latamoxef.

5. The drug form of Claim 2 wherein said β-lactam antibiotic is N-formimidoyl thienamycin monohydrate.

6. The drug form of anyone of Claims 1 to 5 wherein said adjuvant is 5-methoxysalicylic acid, salicylic acid, homovanillic acid; 2,5-dihydroxybenzoic acid; 2,4-dihyroxybenzoic acid; 3,4-dihydroxymandelic acid; 5-methoxy-2-hydroxyphenylsulfonic acid; 3-methylsalicylic acid; 5-methylsalicylic acid; 5-tert-octylsalicylic acid; 3-tert-butyl-6-methylsalicylic acid, 3,5-diisopropylsalicylic acid; 3-tert-butyl-5-methylsalicylic acid; guaicolsulfonic acid; 5-bromosalicylic acid; 3,5-dibromosalicylic acid; 5-iodosalicylic acid; 3,5-diiodosalicylic acid; 2-hydroxyphenylacetic acid; 5-trifluoromethyl-2-hydroxybenzoic acid; 3-methoxysalicylic acid; 5-octyloxysalicylic acid; 5-butoxysalicylic acid; 3,4-dihydroxyphenylacetic acid; 5-chlorosalicylic acid; 3,5-dihydroxybenzoic acid; 2-hydroxy-3-methoxybenzoic acid; or the sodium salts thereof.

7. The drug form of anyone of Claims 1 to 5 wherein the adjuvant is salicylic acid or sodium salicylate.

**Claims for the Contracting State: AT**

1. A method for preparing a β-lactam antibiotic, which upon oral administration provides an increased absorption rate into the bloodstream, by mixing a therapeutically effective dosage amount of the β-lactam antibiotic with an adjuvant having the following formula I

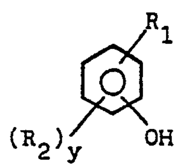

,

wherein

$R_1$ is

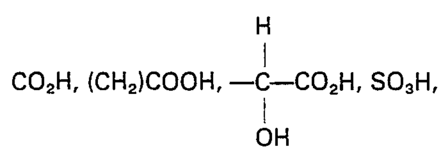

or a pharmaceutically acceptable salt thereof,

$R_2$ is OH, H, an alkoxy radical having 1—10 carbon atoms, an alkyl radical having 1—10 carbon atoms, a halo radical, or a tri-halo alkyl radical, wherein the alkyl group has 1—5 carbon atoms, and

y is an integer of 1 or 2, said adjuvant being present in a sufficient amount to be effective in enhancing said oral absorption rate of said β-lactam antibiotic.

2. The method of Claim 1 wherein said β-lactam antibiotic is a penicillin, a cephalosporin or thienamycin.

3. The method of Claim 2 wherein said penicillin is penicillin G, methacillin, carbenicillin, ticaricillin or amoxicillin.

4. The method of Claim 2 wherein said cephalosporin is cephalosporin C, cefazolin, cephapirin, cephaloridine, cephalothin, cephapirin, cephanone, cefamandole, cefaparole, cefoxitin, cephacetrile, cefmetazole, cefuroxime, cefotaxime, cefoperazone or latamoxef.

5. The method of Claim 1 wherein said β-lactam antibiotic is N-formimidoyl thienamycin monohydrate.

6. The method of anyone of Claims 1 to 5 wherein said adjuvant is 5-methoxysalicylic acid, salicylic acid, homovanillic acid; 2,5-dihydroxybenzoic acid; 2,4-dihyroxybenzoic acid; 3,4-dihydroxymandelic acid;

5-methoxy-2-hydroxyphenylsulfonic acid, 3-methylsalicylic acid; 5-methylsalicylic acid; 5-tert-octylsalicylic acid; 3-tert-butyl-6-methylsalicylic acid; 3,5-diisopropylsalicylic acid; 3-tert-butyl-5-methylsalicylic acid; guaicolsulfonic acid; 5-bromosalicylic acid; 3,5-dibromosalicylic acid; 5-iodosalicylic acid; 3,5-diiodosalicylic acid; 2-hydroxyphenylacetic acid; 5-trifluoromethyl-2-hydroxybenzoic acid; 3-methoxysalicylic acid; 5-octyloxysalicylic acid; 5-butoxysalicylic acid; 3,4-dihydroxyphenylacetic acid; 5-chlorosalicylic acid; 3,5-dihydroxybenzoic acid; 2-hydroxy-3-methoxybenzoic acid; or the sodium salts thereof.

7. The method of anyone of Claims 1 to 5 wherein the adjuvant is salicylic acid or sodium salicylate.

**Patentansprüche für die Vertragsstaaten: BE CH DE FR GB IT LI LU NL SE**

1. Eine oral verabreichbare Arzneimittelform, enthaltend eine therapeutisch wirksame Menge von β-Lactamantibiotikum und ein Adjuvans der folgenden Formel I

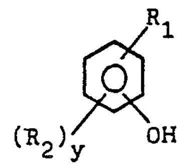,

worin

$R_1$

$$CO_2H,\ (CH_2)COOH,\ -\overset{H}{\underset{OH}{C}}-CO_2H,\ SO_3H$$

oder ein pharmazeutisch annehmbares Salz davon ist,

$R_2$ OH, H, ein Alkoxyrest mit 1 bis 10 Kohlenstoffatomen, ein Alkylrest mit 1 bis 10 Kohlenstoffatomen, ein Halogenrest, oder ein Trihalogenalkylrest, wobei die Alkylgruppe 1 bis 5 Kohlenstoffatomen hat, ist, und

y eine ganze Zahl von 1 oder 2 ist, wobei das genannte Adjuvans in der genannten Arzneimittelform in ausreichender Menge vorliegt, um ein Erhöhen der oralen Absorptionsquote des genannten β-Lactamantibiotikums zu bewirken.

2. Die Arzneimittelform des Anspruchs 1, wobei das genannte β-Lactamantibiotikum ein Penicillin, ein Cephalosporin oder Thienamycin ist.

3. Die Arzneimittelform des Anspruchs 2, wobei das genannte Penicillin Penicillin G, Methacillin, Carbenicillin, Ticaricillin oder Amoxicillin ist.

4. Die Arzneimittelform des Anspruchs 2, wobei das genannte Cephalosporin Cephalosporin C, Cefazolin, Cephapirin, Cephaloridine, Cephalothin, Cephapirin, Cephanone, Cefamandole, Cefaparole, Cefoxitin, Cephacetrile, Cefmetazole, Cefuroxime, Cefotaxime, Cefoperazone oder Latamoxef ist.

5. Die Arzneimittelform des Anspruchs 2, wobei das genannte β-Lactamantibiotikum N-Formimidoylthienamycinmonohydrat ist.

6. Die Arzneimittelform irgendeines der Ansprüche 1 bis 5, wobei das genannte Adjuvans 5-Methoxysalicylsäure, Salicylsäure, Homovanillinsäure; 2,5-Dihydroxybenzoesäure; 2,4-Dihydroxybenzoesäure; 3,4-Dihydroxymandelsäure; 5-Methoxy-2-hydroxyphenylsulfonsäure; 3-Methylsalicylsäure; 5-Methylsalicylsäure; 5-tert.Octylsalicylsäure; 3-tert.Butyl-6-methylsalicylsäure, 3,5-Diisopropylsalicylsäure; 3-tert.Butyl-5-methylsalicylsäure; Guajakolsulfonsäure; 5-Bromsalicylsäure; 3,5-Dibromsalicylsäure; 5-Iodsalicylsäure; 3,5-Diiodsalicylsäure; 2-Hydroxyphenylessigsäure; 5-Trifluormethyl-2-hyroxybenzoesäure; 3-Methoxysalicylsäure; 5-Octyloxysalicylsäure; 5-Butoxysalicylsäure; 3,4-Dihydroxyphenylessigsäure; 5-Chlorsalicylsäure; 3,5-Dihydroxybenzoesäure; 2-Hydroxy-3-methoxybenzoesäure; oder ein Natriumsalz davon ist.

7. Die Arzneimittelform irgendeines der Ansprüche 1 bis 5, wobei das Adjuvans Salicylsäure oder Natriumsalicylat ist.

**Patentansprüche für den Vertragsstaat: AT**

1. Ein Verfahren zur Herstellung eines β-Lactamantibiotikums, welches nach oraler Verabreichung einer erhöhte Absorptionsquote in den Blutstrom bewirkt, durch Vermischen einer therapeutisch wirksamen Dosismenge des β-Lactamantibiotikums mit einem Adjuvans der folgenden Formel I

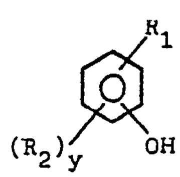,

worin

$R_1$

H

|

$CO_2H$, $(CH_2)COOH$, —C—$CO_2H$, $SO_3H$

|

OH

oder ein pharmazeutisch annehmbares Salz davon ist,

$R_2$ OH, H, ein Alkoxyrest mit 1 bis 10 Kohlenstoffatomen, ein Alkylrest mit 1 bis 10 Kohlenstoffatomen, ein Halogenrest, oder ein Trihalogenalkylrest, wobei die Alkylgruppe 1 bis 5 Kohlenstoffatomen hat, ist, und

y eine ganze Zahl von 1 oder 2 ist, wobei das genannte Adjuvans in ausreichender Menge vorliegt, um ein Erhöhen der genannten oralen Absorptionsquote des genannten β-Lactamantibiotikums zu bewirken.

2. Das Verfahren des Anspruchs 1, wobei das genannte β-Lactamantibiotikum ein Penicillin, ein Cephalosporin oder Thienamycin ist.

3. Das Verfahren des Anspruchs 2, wobei das genannte Penicillin Penicillin G, Methacillin, Carbenicillin, Ticaricillin oder Amoxicillin ist.

4. Das Verfahren des Anspruchs 2, wobei das genannte Cephalosporin Cephalosporin C, Cefazolin, Cephapirin, Cephaloridine, Cephalothin, Cephapirin, Cephanone, Cefamandole, Cefaparole, Cefoxitin, Cephacetrile, Cefmetazole, Cefuroxime, Cefotaxime, Cefoperazone oder Latamoxef ist.

5. Das Verfahren des Anspruchs 1, wobei das genannte β-Lactamantibiotikum N-Formimidoylthienamycinmonohydrat ist.

6. Das Verfahren irgendeines der Ansprüche 1 bis 5, wobei das genannte Adjuvans 5-Methoxysalicylsäure, Salicylsäure, Homovanillinsäure; 2,5-Dihydroxybenzoesäure; 2,4-Dihydroxybenzoesäure; 3,4-Dihydroxymandelsäure; 5-Methoxy-2-hydroxyphenylsulfonsäure; 3-Methylsalicylsäure; 5-Methylsalicylsäure; 5-tert.Octylsalicylsäure; 3-tert.Butyl-6-methylsalicylsäure; 3,5-Diisopropylsalicylsäure; 3-tert.Butyl-5-methylsalicylsäure; Guajakolsulfonsäure; 5-Bromsalicylsäure; 3,5-Dibromsalicylsäure; 5-Iodsalicylsäure; 3,5-Diiodsalicylsäure; 2-Hydroxyphenylessigsäure; 5-Trifluormethyl-2-hyroxybenzoesäure; 3-Methoxysalicylsäure; 5-Octyloxysalicylsäure; 5-Butoxysalicylsäure; 3,4-Dihydroxyphenylessigsäure; 5-Chlorsalicylsäure; 3,5-Dihydroxybenzoesäure; 2-Hydroxy-3-methoxybenzoesäure; oder ein Natriumsalz davon ist.

7. Das Verfahren irgendeines der Ansprüche 1 bis 5, wobei das Adjuvans Salicylsäure oder Natriumsalicylat ist.

**Revendications pour les Etats contractants: BE CH DE FR GB IT LI LU NL SE**

1. Une formulation médicamenteuse pour l'administration orale comprenant une quantité thérapeutique efficace d'antibiotique β-lactame et un adjuvant répondant à la formule I

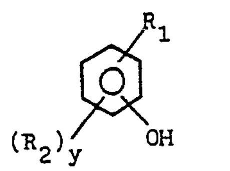

,

dans laquelle

$R_1$ est

H

|

$CO_2H$, $(CH_2)COOH$, —C—$CO_2H$, $SO_3H$,

|

OH

ou un sel pharmaceutiquement acceptable de celui-ci,

$R_2$ est OH, H, un radical alcoxy ayant 1 à 10 atomes de carbone, un radical alkyle ayant 1 à 10 atomes de carbone, un radical halogéno ou un radical trihalogénoalkyle, dans lequel le radical alkyle a 1 à 5 atomes de carbone et

y est un entier de 1 ou 2, ledit adjuvant étant présent dans ladite formulation médicamenteuse en une quantité suffisante pour accroître le taux d'absorption orale dudit antibiotique β-lactame.

2. La formulation médicamenteuse de la revendication 1 dans laquelle ledit antibiotique β-lactame est une pénicilline, une céphalosporine ou la thiénamycine.

3. La formulation médicamenteuse de la revendication 1 dans laquelle ladite pénicilline est la pénicilline G, la méthicilline, la carbénicilline, la ticaricilline ou une amoxicilline.

4. La formulation médicamenteuse de la revendication 1 dans laquelle ladite céphalosporine est la céphalosporine C, la céfazoline, la céfapirine, la céfaloridine, la céfalotine, la céfapirine, la céfénone, le céfamandole, le céfaparole, la céfoxitine, le céfacétrile, le cefmétazole, le céfuroxime, le céfotaxime, la céfopérazone ou le latamoxef.

5. La formulation médicamenteuse de la revendication 2 dans laquelle ledit antibiotique β-lactame est la N-formimidoylthiénamycine monohydratée.

6. La formulation médicamenteuse de l'une quelconque des revendications 1 à 5 dans laquelle ledit adjuvant est l'acide 5-méthoxysalicylique, l'acide salicylique, l'acide homovanillique; l'acide 2,5-dihydroxybenzoïque; l'acide 2,4-dihydroxybenzoïque; l'acide 3,4-dihydroxymandélique; l'acide 5-méthoxy-2-hydroxyphénylsulfonique; l'acide 3-méthylsalicylique; l'acide 5-méthylsalicylique; l'acide 5-tert-octylsalicylique; l'acide 3-tert-butyl-6-méthylsalicylique; l'acide 3,5-diisopropylsalicylique; l'acide 3-tert-butyl-5-méthylsalicylique; l'acide gaïacolsulfonique; l'acide 5-bromosalicylique; l'acide 3,5-dibromosalicylique; l'acide 5-iodosalicylique; l'acide 3,5-diiodosalicylique; l'acide 2-hydroxyphénylacétique; l'acide 5-trifluorométhyl-2-hydroxybenzoïque; l'acide 3-méthoxysalicylique; l'acide 5-octyloxysalicylique; l'acide 5-butoxysalicylique; l'acide 3,4-dihydroxyphénylacétique; l'acide 5-chlorosalicylique; l'acide 3,5-dihydroxybenzoïque; l'acide 2-hydroxy-3-méthoxybenzoïque; ou leurs sels de sodium.

7. La formulation médicamenteuse de l'une quelconque des revendications 1 à 5 dans laquelle l'adjuvant est l'acide salicylique ou le salicylate de sodium.

**Revendications pour l'Etat contractant: AT**

1. Un procédé pour la préparation d'un antibiotique β-lactamine qui, par administration orale, assure un taux d'absorption accru dans la circulation sanguine, par mélange d'une quantité thérapeutiquement efficace de l'antibiotique β-lactame avec un adjuvant répondant à la formule I suivante:

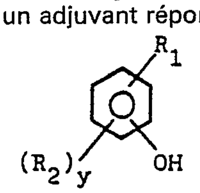
,

dans laquelle

$R_1$ est

$$CO_2H,\ (CH_2)\ COOH,\ -\overset{\displaystyle H}{\underset{\displaystyle OH}{C}}-CO_2H,\ SO_3H,$$

ou un sel pharmaceutiquement acceptable de celui-ci,

$R_2$ est OH, H, un radical alcoxy ayant 1 à 10 atomes de carbone, un radical alkyle ayant 1 à 10 atomes de carbone, un radical halogéno ou un radical trihalogénoalkyle, dans lequel le radical alkyle a 1 à 5 atomes de carbone et

y est un entier de 1 ou 2, ledit adjuvant étant présent en une quantité suffisante pour accroître le taux d'absorption orale dudit antibiotique β-lactamine.

2. Le procédé de la revendication 1 dans lequel ledit antibiotique β-lactame est une pénicilline, une céphalosporine ou la thiénamycine.

3. Le procédé de la revendication 2 dans lequel ladite pénicilline est la pénicilline G, la méthicilline, la carbénicilline, la ticaricilline ou une amoxicilline.

4. Le procédé de la revendication 2 dans lequel ladite céphalosporine est la céphalosporine C, la céfazoline, la céfapirine, la céfaloridine, la céfalotine, la céfapirine, la céfanone, le céfamandole, le céfaparole, la céfoxitine, le céfacétrile, le cefmétazole, le céfuroxime, le céfotaxime, la céfopérazone ou le latamoxef.

5. Le procédé de la revendication 1 dans lequel ledit antibiotique β-lactame est la N-formimidoylthiénamycine monohydratée.

6. Le procédé de l'une quelconque des revendications 1 à 5 dans lequel ledit adjuvant est l'acide 5-méthoxysalicylique, l'acide salicylique, l'acide homovanillique; l'acide 2,5-dihydroxybenzoïque; l'acide 2,4-dihydroxybenzoïque; l'acide 3,4-dihydroxymandélique; l'acide 5-méthoxy-2-hydroxyphénylsulfonique; l'acide 3-méthylsalicylique; l'acide 5-méthylsalicylique; l'acide 5-tert-octylsalicylique; l'acide 3-tert-butyl-6-méthylsalicylique; l'acide 3,5-diisopropylsalicylique; l'acide 3-tert-butyl-5-méthylsalicylique; l'acide gaïacolsulfonique; l'acide 5-bromosalicylique; l'acide 3,5-dibromosalicylique; l'acide 5-iodosalicylique; l'acide 3,5-diiodosalicylique; l'acide 2-hydroxyphénylacétique; l'acide 5-trifluorométhyl-2-hydroxybenzoïque; l'acide 3-méthoxysalicylique; l'acide 5-octyloxysalicylique; l'acide 5-butoxysalicylique; l'acide 3,4-dihydroxyphénylacétique; l'acide 5-chlorosalicylique; l'acide 3,5-dihydroxybenzoïque; l'acide 2-hydroxy-3-méthoxybenzoïque; ou leurs sels de sodium.

7. Le procédé de l'une quelconque des revendications 1 à 5 dans lequel l'adjuvant est l'acide salicylique ou le salicylate de sodium.